# NEUE EUROPÄISCHE PATENTSCHRIFT

(11) **EP 2 632 382 B2**
(45) Veröffentlichungstag und Bekanntmachung des Hinweises auf die Entscheidung über den Einspruch: **26.06.2024**
(45) Hinweis auf die Patenterteilung: 20.09.2017
(21) Anmeldenummer: 11778578.2
(22) Anmeldetag: 28.10.2011
(51) Int. Cl.: A61B 90/00

(54) **NAVIGATIONSAUFSATZ FÜR OPTISCHE GERÄTE IN DER MEDIZIN UND VERFAHREN**
NAVIGATING ATTACHMENT FOR OPTICAL DEVICES IN MEDICINE, AND METHOD
ACCESSOIRE DE NAVIGATION POUR APPAREILS OPTIQUES EN MÉDECINE ET PROCÉDÉ ASSOCIÉ

(30) Priorität: 28.10.2010 DE 102010049702
(43) Veröffentlichungstag der Anmeldung: 04.09.2013
(73) Patentinhaber: Intersect ENT International GmbH, 16761 Hennigsdorf (DE)
(72) Erfinder: ROSE, Andreas, 16565 Oberkrämer (DE); KRÜGER, Timo, 13465 Berlin (DE)
(74) Vertreter: Eisenführ Speiser
(86) Internationale Anmeldenummer: PCT/EP2011/069065
(87) Internationale Veröffentlichungsnummer: WO 2012/056034

(56) Entgegenhaltungen:
- WO-A1-96/05768
- WO-A1-2006/095027
- WO-A1-2007/115825
- WO-A1-2008/076079
- WO-A1-2008/095068
- CHEN JING et al.: "Navigation System for Endoscopic Sinus Surgery Based on Augmented Reality", IEEE /ICCME International Conference on Complex Medical Engineering, 2007, pages 185-188, Beijing
- ICME INTERNATIONAL C ONFERENCE ON, IEEE, PI, 1. Mai2007 (2007-05-01), Seiten 185 188 ISBN: 978-1-4244-1077-4
- Clinical User Guide" Revision 1.2, VectorVision cranial/ENT.Version 7.8, mit Copyright 2008 und nachweisbar veröffentlicht im Jahr 2008 im Rahmen einer offenkundigen Vorbenutzung durch Brainlab (vgl. 01 bis 06 und die folgenden Ausführungenin diesem Abschnitt)

## Beschreibung

Die Erfindung betrifft ein Verfahren sowie eine Vorrichtung zum Darstellen von Bilddaten, bei dem ein optisches Gerät die Lage erfasst und so Lageinformationen gewonnen werden und mit Hilfe der Lageinformationen Bilddaten bestimmt und angezeigt oder weiterverarbeitet werden.

Unter Lageinformation wird hier die Position und die Ausrichtung des optischen Gerätes in Bezug auf ein Referenzkoordinatensystem, also einem Koordinatensystem einer Lageerfassungseinrichtung verstanden. Die Lageinformation kann mittels einer Lageerfassungseinrichtung mit einem entsprechenden Messsystem erfasst werden.

Bei vielen chirurgischen Eingriffen existiert für den Chirurgen das Problem der Orientierung im Patienten während der Operation. Als Planungsgrundlage werden oft Bilddaten des Patienten verwendet, z. B. Computertomographieaufnahmen. Während der Operation arbeitet der Arzt mit direktem Blick auf das Operationsgebiet. Dazu stehen ihm optische Visualisierungssysteme/optische Geräte wie Endoskop oder Mikroskop zur Verfügung. Der Arzt muss zur Umsetzung seiner Planung die Verknüpfung zwischen den präoperativen Bilddaten und der intraoperativen visuellen Information herstellten. Dies kann aufgrund von Orientierungsproblemen zur Verlängerung der Operationsdauer oder zu Fehlern bei der Operation führen.

Als Orientierungshilfe während der Operation sind medizinische Navigationssysteme bekannt. Diese Systeme erfassen während der Operation die Koordinatentransformation zwischen dem Patienten und einem oder mehreren Instrumenten und visualisieren diese in den präoperativen Planungsdaten. Dazu werden Patient und Instrument mit Lokalisatoren ausgestattet, deren Position und Ausrichtung von einem Messsystem als Lageerfassungseinrichtung erfasst werden. Als Messsysteme für eine Lageerfassungseinrichtung werden u. a. elektromagnetische, optische oder Ultraschallsysteme mit entsprechenden Sensoren eingesetzt, wie dies auch für die erfindungsgemäße Vorrichtung in bestimmten Ausführungsformen vorgesehen ist. Die erfindungsgemäße Vorrichtung kann entsprechend ausgestaltet sein. Sie kann entsprechende Einrichtungen hierfür aufweisen.

Es ist bekannt, unterschiedliche Instrumente mit Lokalisatoren für eines solcher Messsysteme zu versehen, z.B. Zeigeinstrument, Sauger, Zange, Nadeln u. ä. und dieses Instrument einzumessen, so dass eine Koordinatentransformation zu einem Bezugspunkt (i. d. R. die Instrumentenspitze) bekannt ist. Die Position des Bezugspunktes bezüglich der Anatomie des Patienten wird in radiologischen Bilddaten des Patienten auf einem Monitor während der Operation angezeigt.

Es ist ferner bekannt neben den Instrumenten, die direkt auf eine Struktur gehalten werden, auch ein optisches Hilfsmittel, wie Endoskop, mit Lokalisatoren und einer Vorrichtung zur Distanzmessung zu versehen und einzumessen. Dadurch wird durch das Navigationssystem die Lage des Endoskops erfasst und durch die Vorrichtung zur Distanzmessung wird die Distanz zu einer Struktur von einem Bezugspunkt des Endoskops erfasst. Die Kombination der beiden Informationen erlaubt die Berechnung und Darstellung der Position des berührungslos erfassten Messpunktes in den Bilddaten.

Es ist bekannt, dass Spulen mit Weicheisenkern als Lokalisatoren in elektromagnetischen Messsystemen mit alternierenden Feldern verwendet werden können. In diesen Spulen werden im wechselnden Feld des sogenannten Feldgenerators des Messsystems charakteristische Spannungen induziert, aus denen die Position der Spule bezüglich des Feldgenerators ermittelt werden kann. Da derartige Spulen als Sensoren in einem elektromagnetischen Messsystem zur Lageerfassung genutzt werden, werden sie auch als Sensorspulen bezeichnet.

Bei der Darstellung der Position der navigierten Instrumente werden oft Schnittbilder aus einem Volumendatensatz (z. B. computertomografisch gewonnen) verwendet und die Position als Punkt, Kreuz o. Ä. in die Schicht eingezeichnet. Oft werden drei orthogonale Schichten angezeigt.

Die bekannten Lokalisatoren vieler Messsysteme müssen aufgrund der Baugröße oder aufgrund des Messprinzips an einem Teil eines Instrumentes, dessen Lage erfasst werden soll, angebracht werden, der außerhalb des Patienten bleibt. Dadurch muss das Instrument starr ausgeführt sein, um eine eindeutige Transformation für die Navigation zu gewährleisten. Flexible Instrumente können also mit einer Positionsmesstechnik, die nicht an der Spitze des Instrumentes angebracht werden können, nicht navigiert werden. Gründe für die Anbringung außerhalb des Patienten können das verwendete Messverfahren, Baugröße der verwendeten Sensorik, unzureichende Ergonomie durch Anbringung der Sensorik oder mangelnde Hygiene sein.

Verfahren zur Darstellung von Bilddaten, bei dem ein optisches Gerät lageerfasst und so Lageinformation gewonnen wird und mit Hilfe der Lageinformation Bilddaten bestimmt und angezeigt oder weiterverarbeitet werden, sind beispielsweise aus den Druckschriften WO 2007/115825 A1, WO 2008/076079 A1 und WO 2008/095068 A1 bekannt.

Dabei ist in der Druckschrift WO 2007/115825 A1 ein Verfahren beschrieben, bei dem reale Bilddaten mittels eines optischen Gerätes aufgenommen werden, gleichzeitig die Position und/oder Ausrichtung des optischen Gerätes erfasst und hieraus eine Lageinformation abgeleitet wird und die von dem optischen Gerät aufgenommenen, realen Bilddaten unter Einbeziehung der Lageinformation zu virtuellen Bilddaten und/oder zusammen mit den virtuellen Bilddaten zu anzuzeigenden Bilddaten verarbeitet werden.

Aufgabe der Erfindung ist es, eine einfache Vorrichtung zur Navigation einer optischen Visualisierungsvorrichtung oder eines optischen Gerätes und ein Verfahren zur Registrierung und/oder Anzeige von Patientenbilddaten zu dieser optischen Visualisierungsvorrichtung anzugeben.

Erfindungsgemäß wird diese Aufgabe zum einen durch ein Verfahren zum Darstellen von Bilddaten gemäß Anspruch 1 gelöst, zum anderen durch eine Vorrichtung zum Darstellen von Bilddaten gemäß Anspruch 8, und schließlich auch durch eine medizinische Behandlungsvorrichtung gemäß Anspruch 10, ein digitales Speichermedium gemäß Anspruch 11, ein Computerprogrammprodukt gemäß Anspruch 12 und ein Computerprogramm gemäß Anspruch 13 gelöst.

Somit werden entweder aus den realen Bilddaten virtuelle Bilddaten generiert, beispielsweise aus realen Bilddaten ein dreidimensionales Modell eines Objekts oder Körperteils erstellt, oder es werden reale, also vom optischen Gerät erfasste Bilddaten mit zuvor generierten virtuellen Bilddaten des jeweiligen Objekts oder Körperteils verknüpft, indem beispielsweise reale Bilddaten in aus den virtuellen Bilddaten abgeleitete Darstellungen eingeblendet werden oder umgekehrt durch virtuelle Bilddaten repräsentierte Strukturen in abgebildete reale Bilddaten eingeblendet werden. Genauso kann auch beides der Fall sein, nämlich dass aus den realen Bilddaten virtuelle Bilddaten generiert und reale Bilddaten in Kombination mit virtuellen Bilddaten zu abzubildenden Bilddaten verarbeitet werden.

Unter virtuellen Bilddaten werden hier solche Bilddaten verstanden, die nicht unmittelbar von dem optischen Gerät aufgenommen werden, sondern beispielsweise bereits vorher mittels eines tomografischen Verfahrens generiert wurden oder in Form eines dreidimensionalen Modells eines jeweiligen Objekts oder Körperteils vorliegen. Vorzugsweise liegen die virtuellen Bilddaten in Form eines Volumendatensatzes vor, in dem die Bilddaten Koordinaten eines Volumens, d.h. eines virtuellen Raum zugeordnet abgespeichert sind.

Vorzugsweise werden darzustellende Bilddaten aus den virtuellen Bilddaten unter Berücksichtigung der Blickrichtung des optischen Gerätes bestimmt, d.h. es werden beispielsweise solche durch die virtuellen Bilddaten repräsentierte Strukturen als darzustellende Bilddaten angezeigt, die in Blickrichtung des optischen Gerätes liegen.

Das Verarbeiten der Bilddaten kann eine Auswahl von anzuzeigenden Bilddaten und/oder eine Transformation der Bilddaten einschließen. Eine Auswahl von Bilddaten ist erforderlich, wenn die virtuellen Bilddaten beispielsweise ein komplettes Körperteil repräsentieren, aber nur die in Blickrichtung des optischen Gerätes liegenden Bilddaten angezeigt werden sollen. Eine Transformation von Bilddaten ist im Weiteren erforderlich, wenn eine Kombination eines realen Objekts oder Körperteils, in dem sich das optische Gerät befindet, mit einem Koordinatensystem in Einklang gebracht werden soll, das dem virtuellen Bilddatensatz zugrunde liegt. Eine Transformation ist auch dann erforderlich, wenn durch einen virtuellen Bilddatensatz repräsentierte Strukturen perspektivrichtig so dargestellt werden sollen, wie es den Abbildungseigenschaften des optischen Gerätes mit seiner jeweiligen aktuellen Position und Ausrichtung entspricht.

Erfindungsgemäß ist das optische Gerät ein Endoskop. Optische Geräte sind dabei solche Geräte, die optische Bilder aufnehmen, wobei diese Bilder nicht notwendigerweise im sichtbaren Wellenlängenbereich aufgenommen zu werden brauchen. Beispielsweise bietet es sich zum Erfassen realer Bilddaten in Blutgefäßen an, reale Bilddaten mit Hilfe von Infrarotlicht in einem Wellenlängenbereich zu gewinnen, in dem Blut eine hohe Transparenz besitzt.

Gemäß einer bevorzugten Ausführungsvariante des Verfahrens schließt das Verfahren der aufgenommenen realen Bilddaten eine photogrammetrische Auswertung der realen Bilddaten zur Gewinnung eines dreidimensionalen Modells des Objekts oder Körperteils ein. Auf diese Weise können aus realen Bilddaten Volumendatensätze gewonnen werden. Bevorzugte Ausführungsdetails dieses Verfahrens sind an anderer Stelle in diesem Text näher erläutert.

Zusätzlich oder alternativ kann das Verfahren so ausgestaltet sein, dass das Verarbeiten der Bilddaten ein Einblenden von virtuellen Bilddaten oder von durch virtuelle Bilddaten repräsentierte Strukturen, Merkmale, Markierungen oder dergleichen in die jeweiligen durch reale Bilddaten repräsentierten Bilder einschließt. Dabei ist es besonders bevorzugt, wenn in reale Bilddaten Konturen von Objekt- oder Körperstrukturen eingeblendet werden, die durch virtuelle Bilddaten repräsentiert sind. Als Beispiel ist hier an anderer Stelle des Textes das Darstellen von einem Hohlgefäß abzweigenden Gefäßen beschrieben.

Auch kann das Verfahren ganz allgemein die Auswahl und Verarbeitung virtueller Bilddaten zu anzuzeigenden Bilddaten derart einschließen, dass die anzuzeigenden Bilddaten Strukturgrenzen eines Objekts oder Körperteils wiedergeben. Solche Strukturgrenzen werden in virtuellen Bilddaten unter Berücksichtigung der Lageinformation für das optische Geräte vorzugsweise dadurch aufgefunden, dass in Blickrichtung des optischen Gerätes verlaufende Vektoren durch einen von den virtuellen Bilddaten beispielsweise in Form eines Volumendatensatzes repräsentierten Raum gelegt und ein Dichtegradient entlang eines jeweiligen Vektors analysiert wird. Ein hoher Dichtegradient, also ein schneller Hell-Dunkel- oder Dunkel-Hell-Übergang werden dabei jeweils als Strukturgrenze des jeweils repräsentierten Objekts oder Körperteils interpretiert. Allgemein bedeutet ein hoher Dichtegradient eine relative starke Veränderung einer durch die virtuellen Bilddaten repräsentierten Größe (wie z.B. Bildhelligkeit) in einem engen Raumabschnitt oder auf einem kurzen Streckenabschnitt eines beispielsweise durch einen Volumendatensatz repräsentierten virtuellen Raums.

Eine erfindungsgemäße Vorrichtung zum Darstellen von Bilddaten umfasst ein optisches Gerät, eine Lageerfassungseinrichtung zum Erfassen der Lage des optischen Gerätes, eine mit dem optischen Gerät und der Lageerfassungseinrichtung verbundene Bilddatenverarbeitungseinrichtung und eine mit der Bilddatenverarbeitungseinrichtung verbundene Anzeigeeinheit. Die Bilddatenverarbeitungseinrichtung ist dabei vorzugsweise so konfiguriert, dass sie ein Verfahren gemäß einem oder mehreren der Ansprüche 1 bis 7 ausführt.

Vorzugsweise weist die Bilddatenverarbeitungseinrichtung einen Speicher für virtuelle Bilddaten auf, dadurch ist die Bilddatenverarbeitungseinrichtung in der Lage, unmittelbar auf virtuelle Bilddaten zurückzugreifen, die im Rahmen des erfindungsgemäßen Verfahrens benötigt werden.

Erfindungsgemäß ist auch eine medizintechnische Behandlungsvorrichtung vorgesehen, die mit einer Vorrichtung gemäß einem der Ansprüche 8 bis 9 verbunden ist und entsprechend vorzugsweise im Rahmen eines Verfahrens gemäß der Ansprüche 1 bis 7 zu benutzen ist.

Ebenso ist erfindungsgemäß ein digitales Speichermedium vorgesehen, beispielsweise in Form einer Diskette, einer CD, einer DVD oder eines anderen an sich bekannten Speichermediums, wobei das Speichermedium elektrisch auslesbare Steuersignale enthält, die so konfiguriert sind, dass das digitale Speichermedium derart mit einem programmierbaren Computersystem zusammenwirkt, dass durch maschinelle Schritte ein erfindungsgemäßes Verfahren gemäß einem der Ansprüche 1 bis 7 veranlasst wird.

Im gleichen Sinne ist erfindungsgemäß auch ein Computerprogrammprodukt vorgesehen, das einen auf einen maschinenlesbaren Träger gespeicherten Programmcode zur Veranlassung der maschinellen Schritte des erfindungsgemäßen Verfahrens gemäß einem der Ansprüche 1 bis 7 aufweist, wenn das Computerprogrammprodukt auf einem Rechner abläuft. Schließlich ist auch ein Computerprogramm in diesem Sinne erfindungsgemäß vorgesehen.

Weitere vorteilhafte Ausgestaltungen des Verfahrens der Vorrichtung sind die Folgenden:
Ein erfindungsgemäßes digitales Speichermedium, insbesondere in Form einer Diskette, CD oder DVD mit elektrisch auslesbaren Steuersignalen, kann derart mit einem programmierbaren Computersystem zusammenwirken, dass die maschinellen Schritte eines erfindungsgemäßen Verfahrens veranlasst werden.

Dabei können alle, einige oder manche der maschinell durchgeführten Schritte des erfindungsgemäßen Verfahrens veranlasst werden.

Ein erfindungsgemäßes Computer-Programm-Produkt weist einen auf einem maschinenlesbaren Träger gespeicherten Programmcode zur Veranlassung der maschinellen Schritte des erfindungsgemäßen Verfahrens, wenn das Computer-Programm- Produkt auf einem Rechner abläuft, auf.

Erfindungsgemäße Weiterbildungen sind jeweils Gegenstand der Unteransprüche und Ausführungsformen.

Ein auch selbstständig und unabhängig von der Darstellung von Bilddaten schutzfähiger Aspekt betrifft eine Vorrichtung für die Navigation - oder genauer: Lageerfassung - des optischen Geräts, die beispielsweise aus einem Lokalisatorträger, insb. einem Spulenträger besteht oder wenigstens einen solchen aufweist.

Der Lokalisatorträger hat vorzugsweise einen an das Gerät angepassten Querschnitt, ist in bestimmten Ausführungsformen hohl und hat vorzugsweise eine geringe Wandstärke.

Der Lokalisatorträger hat in einigen erfindungsgemäßen Ausführungsformen einen passenden Innendurchmesser, so dass er über das Gerät gestülpt und wieder abgezogen werden kann. Vorteilhaft geschieht dies, ohne ein Werkzeug zu verwenden.

Um den Lokalisatorträger als Spulenträger werden in bestimmten Ausführungsformen Hohlspulen gewickelt. Die Abmessungen der Spulen und der Drahtdurchmesser bestimmen die Induktivität der Spule.

Die Induktivität bestimmt die Sensitivität im Messsystem. Die Spulen werden über Drähte eines Kabels, die entlang dem Spulenträger verlegt sind kontaktiert. Drahtlose Ausführungen sind ebenfalls von der vorliegenden Erfindung umfasst.

Über die Spulen - oder erfindungsgemäß anders ausgestaltete Lokalisatoren - und die Drähte (soweit vorhanden) ist zur Kapselung von der Umgebung ein Überzug aufgebracht oder gezogen.

"Spulen" sind hierin als nicht beschränkende Beispiele für Lokalisatoren oder Sensorspulen zu verstehen. Ein Spulenträger ist daher als ein nicht beschränkendes, spezielles Beispiel eines erfindungsgemäßen Lokalisatorträgers zu verstehen.

Der Überzug hat einen geeigneten, vorzugsweise hohlen runden, Querschnitt. Erweist in manchen Ausführungsformen eine geringe Wandstärke auf. Spulen und Überzug sind mit dem Spulenträger dauerhaft verbunden, derart, dass die Spulen und der Zwischenraum von außen gekapselt sind. Dies ist vorteilhaft für die hygienische Aufbereitung der Vorrichtung.

Die optischen Visualisierungssysteme/optischen Geräte werden nachfolgend auch mit dem speziellen Begriff Endoskop beschrieben. Optische Geräte sind erfindungsgemäß Endoskope im engeren Sinne.

Vorteilhafte Weiterentwicklungen des Verfahrens schließen Navigationsaufsätze für alle Arten von Systemen und insbesondere optischen Geräten ein, mit denen der Arzt während der Operation auf oder in das Operationsgebiet schaut (z. B. Laryngoskop, Gastroskop aber auch Mikroskop, etc.).

Der Lokalisatorträger stellt einen einfach nutzbaren Aufsatz für u. a. Endoskope dar. Dieser muss in bestimmten Ausführungsformen nur aufgeschoben werden, um ein z. B. das Endoskop zu einem navigierten (d.h. mit Lageerfassungsvorrichtung bzw. Lokalisator ausgestattetem) Instrument zu erweitern.

In manchen Ausführungsformen befinden sich die Lokalisatoren konzentrisch um das Instrument angeordnet, so dass die Instrumentenachse ohne Einmessung und ohne Dekalibrierungseffekte erfasst wird.

Ein Lokalisator (z.B. die Spule auf dem Spulenträger) kann sich direkt an der Instrumentenspitze befinden. Die Vorrichtung erlaubt eine kompaktere Gestaltung der Lokalisatoren an der Spitze im Vergleich zu allen anderen bekannten Lokalisatoren. Das Instrument mit Navigationsaufsatz ist in manchen Ausführungsformen nur minimal dicker.

In weiteren vorteilhaften Ausführungen dient ein Rohr oder eine Hülse (im Querschnitt geschlossen oder teilweise offen) als Spulenträger (oder ganz allgemein als Lokalisatorenträger). Das Rohr kann z. B. aus Metall oder Kunststoff sein.

Der Überzug kann z. B. ein Rohr sein. Das Rohr kann z. B. aus Metall oder Kunststoff sein.

Der Überzug und /oder der Spulenträger kann z. B. eine Beschichtung sein, die z. B. mit den, dem Fachmann bekannten, Verfahren CVD, PVD, Wirbelsinter etc. erzeugt werden.

Der Überzug und/oder der Spulenträger kann z. B. ein temperaturformbares Material sein (z. B. Schrumpfschlauch).

Der Spulenträger und/oder Überzieher können beide oder einzeln aus einem flexiblen Material sein (z. B. Schlauch).

Erfindungsgemäß können auf dem Spulenträger eine oder mehrere Spulen aufgebracht werden. In bestimmten Ausführungsformen stimmen die Flächennormale der Spulenöffnung nicht mit ihrer Achse des Instruments oder der Achse des Spulenträgers überein. Dadurch ist es vorteilhaft möglich mit der Spule messtechnisch einen Vektor zu erfassen, der nicht kollinear zur Spulenachse und oder zur Spulenträgerachse ist.

Als Spulenträger oder Lokalisatorenträger kann bereits auch die Oberfläche des optischen Geräts verwendet werden.

Der Aufsatz kann in einer vorteilhaften Ausführung am optischen Gerät arretiert werden. Der Aufsatz kann beispielsweise Standardverbindungselemente haben, deren Gegenstück am Instrument ist (z. B. Luer-Lock-Verbindung).

Der Aufsatz kann ein einer vorteilhaften Ausführungsform für andere Instrumente neben optischen Geräten verwendet werden. Z. B. vorteilhaft ist die Verwendung für Sauger oder Katheter mit Führungskanal.

Mit Hilfe der Vorrichtung, d.h. dem Lokalisatorträger oder Aufsatz, wird ein jeweiliges optisches Visualisierungssystem/optisches Gerät auf einfache, vorteilhafte Weise zu einem navigierten optischen Instrument. Dadurch können, wie in der Navigation üblich, Visualisierungen von Instrumenten Positionen in virtuellen Bilddaten möglich werden. Bekannt ist die Darstellung eines navigierten Instrumentes in Schnitten des Patienten(Datensatzes) in einem Patientenkoordinatensystem oder einem Koordinatensystem, das durch das navigierte Instrument gebildet wird.

Das Verfahren der Erfindung erlaubt in manchen erfindungsgemäßen Ausführungsformen vorteilhaft, Visualisierungen an das Operationsverfahren anzupassen und mit Hilfe der Bildinformation des optischen Systems neue Visualisierungsmöglichkeiten zu schaffen.

In den virtuellen Bilddaten, d.h. in dem durch virtuellen Bilddaten repräsentierten virtuellen Raum, wird die Navigationsinformation durch folgendes Verfahren visualisiert. Die Navigationsinformation wird dem Nutzer durch ein Rechenverfahren zur Verfügung gestellt, dass die Achse des vordersten Segmentes des Instrumentes in "Blickrichtung" virtuell verlängert. Entlang dieses so entstehenden Vektors (auch als Blickvektor bezeichnet) wird ein erster Schnittpunkt mit den virtuellen Bilddaten des Patienten berechnet und somit im virtuellen Bilddatensatz der Punkt gefunden und visualisiert, auf den der Operateur schaut.

Der Schnittpunkt wird aus dem Gradienten der Bildpunkte des virtuellen Datensatzes entlang des entstehenden Vektors gefunden. Beim Übergang von Luft zu Gewebe tritt ein starker Gradient auf (Helligkeitsunterschied). Ebenso kann der Übergang von Weichgewebe zu Knochen z. B. durch einen Gradientenschwellenwert zugeordnet werden.

In einer vorteilhaften Ausführung der Erfindung werden mehrere Vektoren zur Detektion mehrerer Punkte oder Linien oder Flächen oder Objekte, die nicht im Zentrum des Endoskops liegen verwendet. Es kann z. B. ein Strahlenbündel mit zwei bis endlich vielen virtuellen Strahlen (Blickvektoren) verwendet werden, die z. B. einen Kegel, vom Endoskop ausgehend, ähnlich dem Sichtkegel, bilden.

Die Strahlen (Blickvektoren) können auch alle in einer Ebene liegen.

Die Schnittpunkte der virtuellen Strahlen des Endoskops mit den virtuellen Bilddaten im virtuellen Raum werden mittels z. B. Gradientenbetrachtung berechnet. Die Visualisierung kann vorteilhaft in verschiedenen crosssektionalen Schnittbildern erfolgen, so dass dem Operateur die Übersicht über alle Grenzschichten des Objektes im Strahlenkegel hat.

Die Schnittbilder (insbesondere die drei orthogonal zueinander stehenden Schnitte) können erfindungsgemäß jeweils zur Strahlennormalen ausgerichtet sein.

Die Schnittbilder (insbesondere die drei orthogonal zueinander stehenden Schnitte) können alle zur Achse des Instruments ausgerichtet sein.

Die Schnittbilder (insbesondere die drei orthogonal zueinander stehenden Schnitte) können alle zur Koordinatendefinition des Models ausgerichtet sein.

Es kann ein Schichtbild in der Ebene des Strahls angezeigt werden und die Strahlen als Linien eingezeichnet werden.

Die Visualisierung der gefundenen Linien kann in bekannten Schnittbildern erfolgen, (2,5 D Darstellung).

Die Darstellung kann als 3D-Modell der erfassten Strukturen erfolgen.

In einer weiteren Ausführung können Objekte sondiert werden. Dabei wird die Tiefeninformation (d.h. der Abstand des virtuellen Strahlursprungs zum Objekt - z.B. repräsentiert durch eine Grenzschicht, die sich in den virtuellen Bilddaten durch einen hohen Dichtegradienten manifestiert - entlang des Strahls) der einzelnen Strahlen verwendet, um Objekte zu identifizieren. Liegt die Tiefeninformation benachbarter Strahlen dicht beieinander, so treffen die Strahlen auf ein Objekt. Ist der Abstand groß, so liegt der eine Strahl auf einem Objekt und der andere liegt auf einem anderen Objekt. Somit werden Objektgrenzen erkannt. Damit können Objekte im virtuellen Modell identifiziert werden.

Im Sinne der zuvor beschriebenen Visualisierung virtueller Bilddaten kann die tiefeninfonation auch als Länge des Blickvektors von seinem Ursprung bis zum "Schnittpunkt", d.h. bis zu dem Ort an dem ein Dichtegradient oberhalb des Gradientenschwellenwertes vorliegt, verstanden werden.

In einer weiteren Ausführung können Objekte in den realen Bilddaten durch das optische Visualisierungssystem detektiert werden. Dazu werden diese Daten digitalisiert (Endoskopkamera).

Für jedes dieser Objekte kann dann ein Schnittbild generiert und angezeigt werden. Das Schnittbild kann z. B. relativ ausgerichtet sein zum Bilddatensatz, zum Instrument oder zu Hauptträgheitsachse des Objektes selbst. Durch mehrere aufeinander folgende Bilddaten entlang einer Bewegungstrajektorie können Objekt detektiert und differenziert werden. Dazu werden photogrammetrische Verfahren verwendet. Vorstellbar ist, dass Features (z. B. Kanten) in einzelnen Bildern erkannt werden. Durch eine Zuordnung der Features zwischen mehreren Bildern kann aufgrund der verschiedenen Bewegung der Features auf verschiedene Objekte geschlossen werden. Zur Verdeutlichung: ein näher liegendes Objekt verändert seine Größe schneller als ein weiter entferntes Objekt. Diesen identifizierten Objekten kann aufgrund der Navigationsinformationen des navigierten Aufsatzes Lageinformationen zugeordnet werden. Es entsteht ein Objektbasiertes 3D-Modell aus den realen Bilddaten.

In einer weiteren Ausführung können die erkannten Objekte im realen und virtuellen Modell in Beziehung zu einander verwendet werden. Es wird z. B. das virtuelle Modell aus dem Vergleich der zugeordneten Objekte angepasst. Fehlt aufgrund des Operationsfortschritts ein Objekt im realen Modell wird das entsprechende Objekt im virtuellen Modell entfernt (intraoperative Modellanpassung). Ist ein Objekt in beiden Modellen vorhanden jedoch zueinander verschieden, kann die Lage des virtuellen Modells mit Hilfe dieser Transformationsinformation angepasst werden (Adaptive Patientenregistrierung).

Ist zu erwarten, dass sich die realen Objekte im Raum zu einander bewegen können (Weichgewebe), kann aus der Lageabweichung der Modelle eine Weichteilbewegung erkannt werden. Das virtuelle Modell kann dementsprechend angepasst werden.

Das navigierte Instrument, also beispielsweise das optische Gerät mit Lageerfassungseinrichtung, das durch die Verwendung des Aufsatzes entsteht (optisches Visualisierungssystem mit Navigationsaufsatz) wird durch das beschriebene Verfahren für die Registrierung der Patientendaten verwendet. Dabei werden mehrere Objekte in den realen Bilddaten zu virtuellen Objekten zugeordnet. Beispielsweise eignen sich dazu die Nasenöffnungen, Augenhöhlen, Ohrmuscheln. Ein in beiden Modellen bekanntes Objekt muss dabei in den realen Bildern immer sichtbar sein und identifiziert werden. Dazu eignet sich der sogenannte Patientenlokalisator.

Das Verfahren kann auch dazu verwendet werden, um die Transformation des virtuellen Modells zum realen Modell zu bestimmen. Dadurch können beispielsweise Objekte, die im Videobild gar nicht vorhanden sind, z. B. Planungsdaten (für Rekonstruktion, Implantate etc.), die im virtuellen Datensatz geplant sind, im realen Videobild oder in dem realen Modell dargestellt werden. Ebenso können verdeckte Strukturen im realen Videobild (z. B. Abzweige von Adern, Zielregionen) überlagert dargestellt werden, beispielsweise indem die Konturen der verdeckten Strukturen perspektivrichtig in ein reales Bild (beispielsweise mit hellen oder dunklen Linien) "eingezeichnet" werden.

In einer weiteren vorteilhaften Ausführung der Erfindung wird über alle sichtbaren Punkte eine virtuelle Oberflächenabtastung vorgenommen und die Informationen dann auf ein geeignetes Referenzobjekt projiziert, nach Verfahren, die z. B. aus der geographischen Kartenherstellung bekannt sind. Daraus ergibt sich eine neue Art der Darstellung. Das Verfahren ist auch mit Linien als Abwandlung einer zylindrischen Projektion anwendbar. Das geeignete Referenzobjekt kann beispielsweise ein einfaches geometrisches Objekt wie z.B. ein Zylinder sein. Das Referenzobjekt kann aber auch ein virtuelles dreidimensionales Modell eines von einem optischen Gerät erfassten Objekts sein. In beiden Fällen werden vom optischen Gerät erfasste Bilddaten undvirtuelle Bilddaten unter Auswertung der Lageinformation miteinander verknüpft.

Die Visualisierung der detektierten Punkte kann auch im korrespondierenden Videobild eines Videoendoskops erfolgen. Zuvor müssen die Abbildungseigenschaften bei der Aufnahme des Videobildes erfasst worden sein.

Durch die beschriebenen Projektionsverfahren besteht die Möglichkeit einer völlig neuen Informationsdarstellung (z.B. der Tunnelentzerrung). Hier werden geeignete virtuelle Bildpunkte, durch geeignete Vektoranordnungen aus dem Patientenbilddatensatz extrahiert, z. B. kreisförmig oder linienförmig oder vollständig in Gefäßen. Das Ergebnis wird dann auf vordefinierte Zielstrukturen projiziert, z. B. Zylinder, Kugeln, Ellipsoide. Erfindungsgemäß wird somit in manchen Ausführungsformen eine vorteilhafte, also eine an die Operationsweise angepasste Darstellung der Navigationsinformation des navigierten Instruments möglich.

Das erfindungsgemäße Verfahren und seine Varianten sowie die erfindungsgemäße Vorrichtung und weitere vorteilhafte Ausgestaltungen des Verfahrens und der Vorrichtung sind der nachfolgenden Beschreibung eines Ausführungsbeispiels zu entnehmen. Die beigefügten Abbildungen zeigen beispielhaft Aspekte einer erfindungsgemäßen Vorrichtung, nämlich in
- Abb. 1:: eine Bilddatenverarbeitungseinrichtung und eine mit der Bilddatenverarbeitungsvorrichtung verbundene Anzeigeeinheit;
- Abb. 2:: eine Lageerfassungseinrichtung zum Erfassen der Lage des optischen Gerätes;
- Abb. 3:: ein optisches Gerät und die Lageerfassungseinrichtung zum Erfassen der Lage des optischen Gerätes.

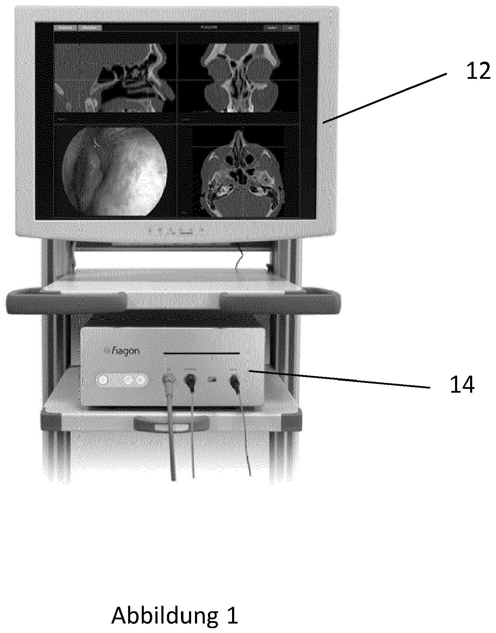

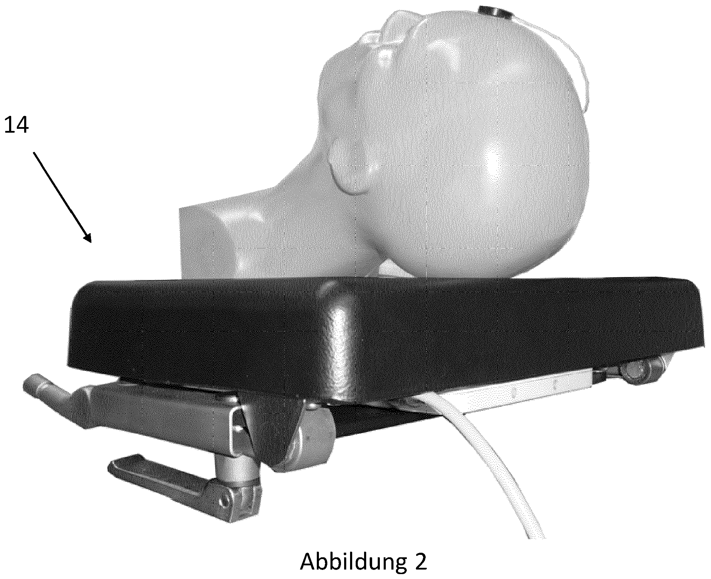

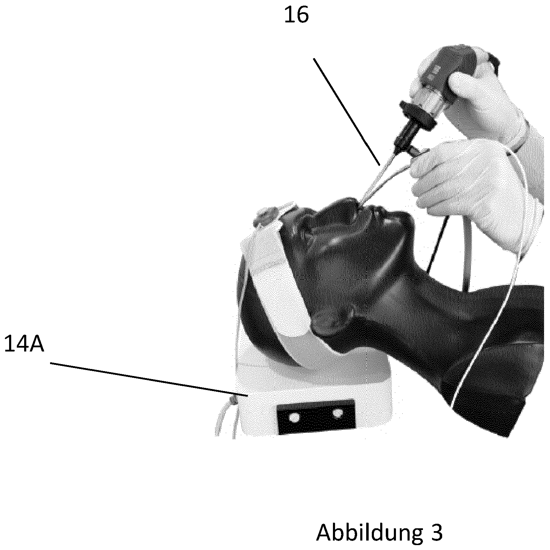

Die Abbildungen 1 bis 3 zeigen die Komponenten einer erfindungsgemäßen Vorrichtung zur Darstellung von Bilddaten. Die Vorrichtung umfasst eine Bildverarbeitungseinrichtung 10, an die ausgangsseitig eine Anzeigeeinheit 12 angeschlossen ist. Eingangsseitig sind an die Bildverarbeitungseinrichtung 10 eine Lageerfassungseinrichtung 14 (siehe Abbildungen 2 und 3) sowie ein optisches Gerät 16 (siehe Abbildung 3), nämlich ein Endoskop, angeschlossen.

Die Lageerfassungseinrichtung kann in sich bekannter Weise einen Feldgenerator 14A für ein elektromagnetisches Wechselfeld aufweisen sowie Sensorspulen, die an dem Gerät befestigt sind, dessen Lage erfasst werden soll. In diesem Falle sind die Sensorspulen nicht erkennbar an dem Endoskop 16 angebracht, und zwar vorzugsweise in der Nähe von dessen distalem Ende. Bevorzugt sind die Sensorspulen klein und besitzen einen weichmagnetischen Kern. Zwei oder drei Sensorspulen sind außermittig in Bezug auf die Längsachse des Endoskopes 16 an dessen distalem Ende angeordnet und weisen eine unterschiedliche Orientierung auf. Auf diese Weise kann in an sich bekannter Weise nicht nur die Lage der einzelnen Spulen in dem von dem Feldgenerator 14A erzeugten elektromagnetischen Wechselfeld erfasst werden, sondern auch deren Orientierung, d.h. Ausrichtung. Auf diese Weise kann mit der Lageerfassungseinrichtung 14 sowohl die Position als auch die Ausrichtung beispielsweise eines distalen Endes des Endoskopes 16 zu einem jeweiligen Zeitpunkt genau bestimmt werden.

Die Sensorspulen sind Teil der Lageerfassungseinrichtung und werden an anderer Stelle in diesem Text auch als Lokalisatoren oder- zusammen mit ihrem Träger - als Lageerfassungsvorrichtung bezeichnet.

Damit diese Lageinformation in Bezug auf einen Patienten 18 aussagekräftig ist, ist eine weitere Sensorspule 14B ortsfest am relevanten Körperteil des Patienten (in diesem Falle dem Kopf) befestigt.

Um virtuelle Bilddaten, also beispielsweise tomografisch gewonnene Schnittbilder des Patientenkopfes, mit den von der Lageerfassungseinrichtung 14 erfassten augenblicklichen Positionen in Einklang zu bringen, wird zunächst eine Registrierung des Körperteils des Patienten - beispielsweise also des Kopfes - vorgenommen, wie dies ebenfalls grundsätzlich bekannt ist. Anschließend kann eine jeweils mit der Lageerfassungseinrichtung 14 gewonnene Lageinformation einen Ort in den virtuellen Bilddaten, also beispielsweise in entsprechenden Tomografien des Patientenkopfes, zugeordnet werden.

Letztes erlaubt eine an sich bekannte Navigation eines Instrumentes durch Darstellung der Instrumentenposition in tomografisch gewonnenen Schnittbildern eines Körperteils.

Abweichend oder ergänzend zu dieser an sich bekannten Navigation erlaubt es die Bildverarbeitungseinrichtung 10, vom optischen Instrument 16 aufgenommene reale Bilddaten und diesen jeweils zugeordnete Lageinformationen gleichzeitig zu erfassen. Dies bedeutet, dass zu einem von dem Endoskop 16 aufgenommenen Einzelbild auch jeweils die Lageinformation aufgenommen wird, die die Position und Ausrichtung des optischen Gerätes 16 für den Augenblick beschreibt, zu dem das jeweilige Einzelbild aufgenommen wurde.

Auf diese Weise ist die Bildverarbeitungseinrichtung beispielsweise in der Lage, vom optischen Gerät 16 aufgenommene reale Bilddaten mit virtuellen Bilddaten zu verknüpfen. Solche virtuellen Bilddaten können tomografisch aufgenommene Bilddaten sein. Die virtuellen Bilddaten können aber auch Daten eines dreidimensionalen Modells des jeweiligen Körperteils sein.

Auf diese Weise ist es beispielsweise möglich, dass die Bildverarbeitungseinrichtung 10 in ein reales, von dem optischen Gerät 10 aufgenommenes Bild, beispielsweise eines länglichen Hohlorgans, Strukturen einzeichnet, die auf dem optisch aufgenommenen Bild sonst nicht zu erkennen wären. Zeigt von dem Hohlorgan, das von dem optisch aufgenommenen Bild dargestellt wird, beispielsweise ein Hohlorgan an versteckter Stelle ab, so können die Konturen dieses Hohlorgans perspektivisch richtig, beispielsweise durch helle oder dunkle Linien, in das optisch aufgenommene Bild eingezeichnet werden. Dies würde es beispielsweise einem Operateur erlauben, ein abzweigendes Gefäß leicht zu finden, obwohl es auf Basis realer, von dem optischen Gerät aufgenommener Bilddaten gar nicht oder nur schwer zu erkennen ist.

In einem alternativen Szenario können mit der erfindungsgemäßen Vorrichtung zum Darstellen von Bilddaten ein 3D-Modell eines Körperteils repräsentierende virtuelle Bilddaten gerade dadurch gewonnen werden, dass von dem optischen Gerät 16 aufgenommene reale Bilddaten unter Berücksichtigung der jeweils zugehörigen Lageinformation bearbeitet werden. Dazu kann das optische Gerät 16 durch beispielsweise ein Hohlorgan hindurchbewegt werden und dabei sukzessive eine Folge von Einzelbildern des Hohlorgans aufnehmen. Da die Einzelbilder von jeweils unterschiedlicher, aber aufgrund der zugehörigen Lageinformation bekannter Position und Ausrichtung des optischen Gerätes 16 aufgenommen wurden, zeigen sie Strukturen des Hohlorgans aus unterschiedlicher Perspektive mit Hilfe in aufeinanderfolgenden Einzelbildern wiedererkennbaren charakteristischen Punkten des Hohlorgans. So kann unter Berücksichtigung der jeweiligen Lageinformation auf photogrammetrischem Wege ein dreidimensionales Modell des Hohlorgans allein auf Basis der realen Bilddaten und der zugehörigen Lageinformation konstruiert werden.

In einem weitergehenden Schritt kann die Bildverarbeitungseinrichtung auch dazu ausgebildet sein, dass ein auf diese Weise intraoperativ gewonnenes dreidimensionales Modell von der Bildverarbeitungseinrichtung 10 dazu herangezogen werden kann, ein durch virtuelle Bilddaten repräsentiertes, zuvor erstelltes dreidimensionales Modell des Körperteils oder Hohlorgans in Echtzeit zu korrigieren und an die tatsächlichen Gegebenheiten anzupassen. Eine vorteilhafte Ausführungsvariante der Bildverarbeitungseinrichtung bleibt somit eine intraoperative Modellanpassung in Echtzeit.

Eine weitere von der Bildverarbeitungseinrichtung 10 je nach Ausführungsvariante mögliche Verarbeitung virtueller und realer, d.h. vom optischen Gerät 16 aufgenommener, Bilddaten besteht darin, dass die realen Bilddaten gewissermaßen auf Wände in diesen virtuellen Bilddaten projiziert werden, so dass auf der Anzeigeeinheit ein dreidimensionales Modell, beispielsweise eines Körperteils oder Hohlorgans, dargestellt werden kann, dessen Oberflächen genauso erscheinen, dass sie den vom optischen Gerät 16 erfassten realen Bilddaten in ihrem Aussehen entsprechen. Somit kann ein vollkommen realistisch aussehendes dreidimensionales Modell des jeweiligen Körperteils oder Hohlorgans auf der Anzeigeeinheit 12 dargestellt werden. Die Anzeigeeinheit 12 kann zu diesem Zweck in besonders vorteilhafter Weise ein 3D-Monitor sein.

## Patentansprüche

1. Verfahren zur Darstellung von Bilddaten, bei dem ein optisches Gerät lageerfasst und so Lageinformation gewonnen wird und mit Hilfe der Lageinformation Bilddaten bestimmt und angezeigt oder weiterverarbeitet werden, wobei die Lageinformation des optischen Geräts mittels eines medizinischen Navigationssystems erfasst wird, das während einer Operation eine Koordinatentransformation zwischen einem Patienten und dem optischen Gerät erfasst und dieses in präoperativen Planungsdaten visualisiert, wobei der Patient und das optische Gerät mit Lokalisatoren ausgestattet sind, deren Position und Ausrichtung von einem Messsystem als Lageerfassungseinrichtung erfasst werden, wobei die anzuzeigenden Bilddaten wenigstens teilweise auf virtuellen, nicht unmittelbar von dem optischen Gerät erfassten Bilddaten beruhen, die ein Objekt oder Körperteil repräsentieren welches sich in einem Blickbereich des optischen Gerätes befindet, wobei im Rahmen des Verfahrens:
- reale Bilddaten mittels eines optischen Gerätes aufgenommen werden,
- gleichzeitig die Position und Ausrichtung des optischen Gerätes erfasst und hieraus eine Lageinformation abgeleitet wird,
- die von dem optischen Gerät aufgenommenen realen Bilddaten unter Einbeziehung der Lageinformation zu virtuellen Bilddaten und/oder zusammen mit den virtuellen Bilddaten zu anzuzeigenden Bilddaten verarbeitet werden,
wobei das optische Gerät eine Blickrichtung hat und darzustellende Bilddaten aus den virtuellen Bilddaten unter Berücksichtigung der Blickrichtung des optischen Gerätes bestimmt werden und wobei verdeckte Strukturen in den realen Bilddaten überlagert dargestellt werden und wobei das obtische Gerät ein Endoskop ist.

2. Verfahren nach Anspruch 1, **dadurch gekennzeichnet, dass** die virtuellen Bilddaten mittels eines tomographischen Verfahrens gewonnen sind und/oder Daten eines dreidimensionalen Modells eines Objekts oder Körperteils sind.

3. Verfahren nach einem der Ansprüche 1 bis 2, **dadurch gekennzeichnet, dass** das Verarbeiten der Bilddaten eine Auswahl von anzuzeigenden Bilddaten und/oder eine Transformation der Bilddaten einschließt.

4. Verfahren nach einem der Ansprüche 1 bis 3, **dadurch gekennzeichnet, dass** das Verarbeiten der aufgenommenen Bilddaten eine photogrammetrische Auswertung der realen Bilddaten zur Gewinnung eines dreidimensionalen Modells des Objekts oder Körperteils einschließt.

5. Verfahren nach einem der Ansprüche 1 bis 4, **dadurch gekennzeichnet, dass** das Verarbeiten der Bilddaten ein Einblenden von virtuellen Bilddaten in die jeweiligen realen Bilddaten einschließt.

6. Verfahren nach Anspruch 5, **dadurch gekennzeichnet, dass** in reale Bilddaten Konturen von Objekt- oder Körperteilstrukturen eingeblendet werden, die durch virtuelle Bilddaten repräsentiert sind.

7. Verfahren nach einem der Ansprüche 1 bis 6, **dadurch gekennzeichnet, dass** anzuzeigende Bilddaten unter Berücksichtigung virtueller Bilddaten in Form von Tomographien und unter Berücksichtigung der Blickrichtung des optischen Gerätes dadurch bestimmt werden, dass der Blickrichtung des optischen Gerätes entsprechende Blickvektoren in den virtuellen Bilddaten derart analysiert werden, dass starker Dichtegradient entlang eines jeweiligen Blickvektors als Strukturgrenze des jeweils repräsentierten Objekts oder Körperteils interpretiert wird.

8. Vorrichtung zur Darstellung von Bilddaten, welche ein optisches Gerät, eine Lageerfassungseinrichtung zum Erfassen der Lage des optischen Gerätes, eine mit dem optischen Gerät und der Lageerfassungseinrichtung verbundene Bilddatenverarbeitungseinrichtung und eine mit der Bilddatenverarbeitungsvorrichtung verbundene Anzeigeeinheit aufweist, wobei die Bilddatenverarbeitungsvorrichtung dazu konfiguriert ist, ein Verfahren gemäß den Ansprüchen 1 bis 7 auszuführen.

9. Vorrichtung nach Anspruch 8, **dadurch gekennzeichnet, dass** die Bilddatenverarbeitungsvorrichtung eine Speicher für virtuelle Bilddaten aufweist.

10. Medizintechnische Behandlungsvorrichtung verbunden mit wenigstens einer Vorrichtung nach Anspruch 8 oder 9 und/oder zum Durchführen wenigstens eines Verfahrens nach einem der Ansprüche 1 bis 7.

11. Digitales Speichermedium, insbesondere in Form einer Diskette, CD oder DVD, mit elektrisch auslesbaren Steuersignalen, konfiguriert, um derart mit einem programmierbaren Computersystem zusammenwirken, dass die maschinellen Schritte eines erfindungsgemäßen Verfahrens nach einem der Ansprüche 1 bis 7 veranlasst werden.

12. Computer-Programm-Produkt mit einem auf einem maschinenlesbaren Träger gespeicherten Programmcode zur Veranlassung der maschinellen Schritte des erfindungsgemäßen Verfahrens gemäß einem der Ansprüche 1 bis 7, wenn das Computer-Programm- Produkt auf einem Rechner abläuft.

13. Computer-Programm mit einem Programmcode zur Veranlassung der maschinellen Schritte eines erfindungsgemäßen Verfahrens gemäß einem der Ansprüche 1 bis 7, wenn das Computer-Programm auf einem Computer abläuft.

## Claims

1. Method for presenting image data, wherein an optical device detects the position and position information is thus obtained and image data are determined and illustrated or processed further using the position information, wherein the position information of the optical device is acquired by means of a medical navigation system, which, during an operation, acquires a co-ordinate transformation between a patient and the optical device and visualises this in pre-operative planning data, wherein the patient and the optical device are fitted with localisers whose position and orientation are acquired by a measuring system as a position acquisition device, wherein the image data to be illustrated involve at least partially virtual image data, not acquired directly by the optical device, said image data representing an object or a body part which is located in a field of vision of the optical device, wherein, within the scope of the method:
- actual image data are recorded by means of the optical device,
- the position and orientation of the optical device are acquired simultaneously, from which position information is derived,
- the actual image data recorded by the optical device are processed taking into account the position information for the virtual image data and/or together with the virtual image data for the image data to be illustrated,
wherein the optical device has a viewing direction and image data to be illustrated are determined from the virtual image data taking into account the viewing direction of the optical device and wherein covered structures are shown superimposed in the actual image data and wherein the optical device is an endoscope.

2. Method according to claim 1, **characterised in that** the virtual image data are obtained by means of a tomographic method and/or data of a three-dimensional model of an object or body part.

3. Method according to any one of claims 1 to 2, **characterised in that** the processing of the image data includes a selection of image data to be illustrated and/or a transformation of the image data.

4. Method according to any one of claims 1 to 3, **characterised in that** the processing of the recorded image data includes a photogrammetric evaluation of the actual image data to obtain a three-dimensional model of the object or body part.

5. Method according to any one of claims 1 to 4, **characterised in that** the processing of the image data includes a fade-in of virtual image data into the particular actual image data.

6. Method according to claim 5, **characterised in that** contours of object or body part structures which are represented by virtual image data are faded into actual image data.

7. Method according to any one of claims 1 to 6, **characterised in that** image data to be illustrated are determined, taking into account virtual image data in the form of tomographic images and taking into account the visual direction of the optical device such that the visual vectors in the virtual image data corresponding to the visual direction of the optical device are analysed such that a strong density gradient is interpreted along a particular visual vector as a structural boundary of the object or body part represented at any given time.

8. Device for presenting image data, which has an optical device, a position acquiring device to acquire the position of the optical device, an image data processing device connected to the optical device and to the position acquiring device and a display unit connected to the image data processing device, wherein the image data processing device is also configured to perform a method in accordance with claims 1 to 7.

9. Device according to claim 8, **characterised in that** the image data processing device has storage for virtual image data.

10. Medical treatment apparatus connected to at least one device according to claim 8 or 9 and/or to perform at least one method according to any one of claims 1 to 7.

11. Digital storage medium, in particular in the form of a disc, CD or DVD, with electrically readable control signals, configured so that it is able to interact with a programmable computer system such that the computerised steps of a method according to the invention are performed according to any one of claims 1 to 7.

12. Computer programme product with a programme code stored on a machinereadable carrier to perform the computerised steps of the method according to the invention according to any one of claims 1 to 7 when the computer programme product runs on a computer.

13. Computer programme with a programme code to perform the computerised steps of a method according to the invention according to any one of claims 1 to 7, when the computer programme runs on a computer.

## Revendications

1. Procédé de représentation de données d'image, dans lequel on détecte en position un appareil optique et on obtient ainsi des informations de position et, à l'aide des informations de position, on détermine des données d'image et on les affiche ou on les retraite, dans lequel on détecte les informations d'image de l'appareil optique au moyen d'un système de navigation de médecine, qui, pendant une opération, détecte une transformation de coordonnées entre un patient et l'appareil optique et la visualise en des données de programme pré-opératives, le patient et l'appareil optique étant équipés de localisateurs, dont la position et la direction sont détectées par un système de mesure sous la forme d'un dispositif de détection de position, les données d'image affichées reposant, au moins en partie, sur des données d'image virtuelles obtenues indirectement par l'appareil optique, qui représentent un objet ou une partie du corps se trouvant dans une zone de visualisation de l'appareil optique, dans lequel, dans le cadre du procédé :
- on enregistre des données d'image réelles de l'appareil optique,
- on détecte en même temps la position et la direction de l'appareil optique et on en déduit une information de position,
- on traite les données d'image réelles enregistrées par l'appareil optique en incorporant l'information de position aux données d'image virtuelles et/ou aux données d'image à afficher ensemble avec les données d'image virtuelles,
dans lequel l'appareil optique a une direction de visualisation et des données d'image à représenter sont déterminées à partir des données d'image virtuelles en tenant compte de la direction de visualisation de l'appareil optique et dans lequel des structures cachées dans les données d'image réelles sont représentés en superposition et dans lequel l'appareil optique est un endoscope.

2. Procédé suivant la revendication 1, **caractérisé en ce que** les données d'image virtuelles sont obtenues au moyen d'un procédé tomographique et/ou sont des données d'un modèle en trois dimensions d'un objet ou d'une partie du corps.

3. Procédé suivant l'une des revendications 1 à 2, **caractérisé en ce que** le traitement des données d'image inclue une sélection de données d'image à afficher et/ou une transformation des données d'image.

4. Procédé suivant l'une des revendications 1 à 3, **caractérisé en ce que** le traitement des données d'image enregistrées inclue une exploitation photogrammétrique des données d'image réelles en obtenant un modèle en trois dimensions de l'objet ou de la partie du corps.

5. Procédé suivant l'une des revendications 1 à 4, **caractérisé en ce que** le traitement des données d'image inclue une incrustation de données d'image virtuelles dans les données d'image réelles.

6. Procédé suivant la revendication 5, **caractérisé en ce que** l'on incruste dans des données d'image réelles des contours de structure d'objet ou de partie du corps, qui sont représentés par des données d'image virtuelles.

7. Procédé suivant l'une des revendications 1 à 6, **caractérisé en ce que** l'on détermine des données d'image à afficher en tenant compte de données d'image virtuelles sous la forme de tomographies et en tenant compte de la direction de visualisation de l'appareil optique, en analysant des vecteurs de visualisation correspondant à la direction de visualisation de l'appareil optique dans les données d'image virtuelles, de manière à interpréter un gradient d'intensité fort le long d'un vecteur de visualisation comme étant une limite de structure de l'objet ou de la partie du corps représentée.

8. Installation de représentation de données d'image, qui a un appareil optique, un dispositif de détection de position pour détecter la position de l'appareil optique, un dispositif de traitement de données d'image relié à l'appareil optique et au dispositif de détection de position et une unité d'affichage reliée au dispositif de traitement de données d'image, le dispositif de traitement de données d'image étant configuré à cet effet pour effectuer un procédé suivant l'une des revendications 1 à 7.

9. Installation suivant la revendication 8, **caractérisée en ce que** le dispositif de traitement de données d'image a une mémoire pour des données d'image virtuelles.

10. Installation de traitement en technique de médecine, prévue pour la liaison ou pour être reliée à au moins une installation suivant la revendication 8 ou 9 et/ou pour effectuer au moins un procédé suivant l'une des revendications 1 à 7.

11. Support de mémoire numérique, notamment sous la forme d'une disquette CD ou DVD, comprenant des signaux de commande pouvant être déchiffrables électriquement, configuré pour coopérer avec un système d'ordinateur programmable, de manière à faire se dérouler les stades de machine d'un procédé suivant l'invention suivant l'une des revendications 1 à 7.

12. Produit de programme d'ordinateur, comprenant un code de programme mémorisé sur un support déchiffrable par ordinateur pour provoquer les stades automatiques du procédé suivant l'invention suivant l'une des revendications 1 à 7, lorsque le produit de programme d'ordinateur passe sur un ordinateur.

13. Programme d'ordinateur ayant un code de programme pour faire se dérouler les stades de machine d'un procédé suivant l'invention suivant l'une des revendications 1 à 7, lorsque le programme d'ordinateur passe
